# EUROPEAN PATENT APPLICATION

(11) **EP 2 263 702 A1**
(43) Date of publication of application: **22.12.2010**
(21) Application number: 09425233.5
(22) Date of filing: 16.06.2009
(51) Int. Cl.: A61L 9/12, B60H 3/00, B65D 1/32

(54) **Deodorant liquid substance dispenser for use in vehicles**

(71) Applicant: New Project di Fiorenzo Cipolla, 20020 Cogliate (MI) (IT)
(72) Inventor: Cipolla, Fiorenzo, 20020 Cogliate (IT)
(74) Representative: Riccardi, Sergio

(57) **Abstract**

A deodorant liquid dispenser (10), particularly suitable for use in vehicles, to supply said deodorant liquid in form of drops to a diffusing element (7). The delivery of the drops occurs through a nebulizer unit (3) fed by the pressure exerted on said liquid during the compression of the bellows container (1) of said liquid.

## Description

The present invention relates to a deodorant substance dispenser, in particular said dispenser is suitable for use within vehicles.

Within the vehicle deodorant market a lot of solutions are available which, according to methods very different from each other, provide for delivery of deodorant substances in order to cover or eliminate smell not pleasant for the car occupants, first of all, the cigarette smoke smell, possible smells coming from the outlets of the air conditioning system, the filter of which can be often soaked by substances being in the atmosphere and having a not pleasant smell.

Such deodorants can be in a solid form, such as sticks, cards just soaked by a particular deodorant substance having any kind of shape or fine deodorant powder to be poured for example into the ashtray within the vehicle, or even in gaseous or liquid state. The latter case, that is the deodorant in a liquid state, requires necessarily the presence of a dispenser in order to supply a body suitable for diffusing said deodorant stored in said dispenser but, on the other hand, they have the clear advantage of a greater length of time in respect of the deodorants in solid state, since the liquid is delivered as the one just delivered to said diffusing body is used up. Several of such dispensers obtain this result, for example, by liquid drops falling by gravity onto the diffusing body, having the drawback, in case of lack of tight seal between dispenser and diffuser, that said liquid drops can leak directly from the dispenser inside the vehicle in which they are used. Other dispenser instead are provided with electronic devices able to automatically dose the deodorant substance delivery: said devices obviously have the consequent drawback of a higher cost in respect of the preceding solution.

Main object of the present invention is therefore to provide a dispenser for a deodorant liquid substance able to soak a body suitable for diffusing the substance without liquid leakage.

Further object of the present invention is even to provide a dispenser for a deodorant liquid substance able to be actuated by user in order to cause said body suitable for diffusion to be soaked by said deodorant substance every time the user consider it necessary.

Further object of the present invention is to provide a deodorant liquid substance dispenser which can be manufactured at a very low cost.

A detailed description of preferred embodiments of a deodorant liquid substance according to the present invention will be now provided, with reference to the annexed drawings, wherein:
fig. 1 is an exploded perspective view of a first preferred embodiment of the deodorant liquid dispenser according to the present invention, and
fig. 2 is an exploded perspective view of a second preferred embodiment of the deodorant liquid dispenser according to the present invention.

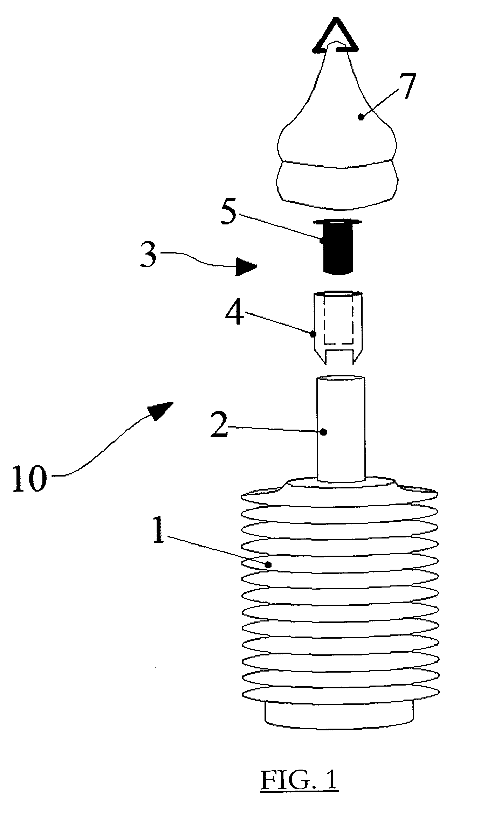

Fig. 1 shows a dispenser 10 comprising a bellows container 1 provided with a cylindrical open end 2. Said cylindrical end serves as housing for a nebulizer unit 3 in turn constituted by a nebulizer 4 and a section reducer 5. Said nebulizer 4 is basically constituted by a conduit having a short longitudinal development and provided with a narrowing section having a size suitable to allow for passage of the deodorant liquid almost exclusively in form of drops. Not limiting, then, for the operation of said dispenser 10 but provided in the disclosed embodiment, there is a further cylindrical body 6 having an inner through chamber with a diameter equal to the diameter of said cylindrical end 2, such that said body 6 can be fitted onto said end 2. Therefore, the nebulizer unit 3 is inserted in said cylindrical end 2 and, as just disclosed, said cylindrical body 6 can be inserted therearound. Finally, even on said cylindrical end 2, there is inserted a diffusing element 7, which, due to the material it is constituted of, is able to absorb the nebulized liquid.

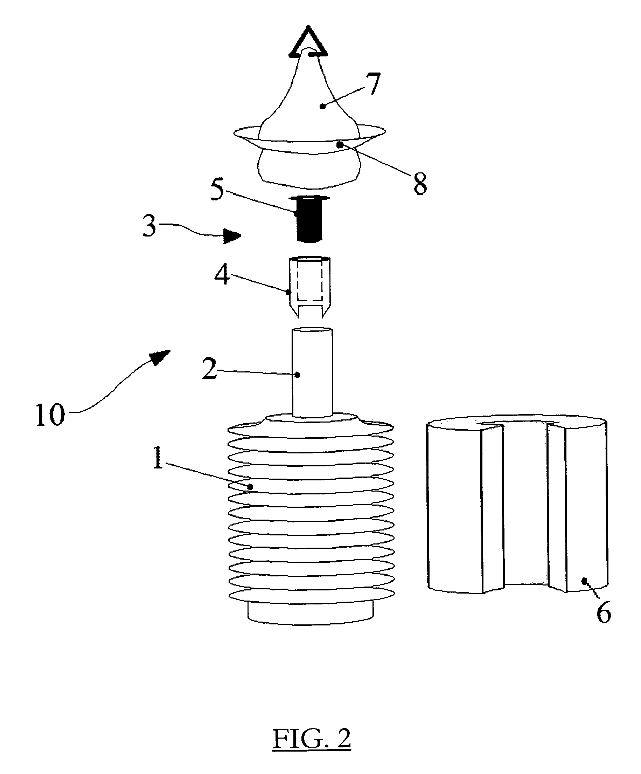

Once mounted, said dispenser 10 is hung, for example, on the rear-view mirror inside the vehicle in which said dispenser is used. If it is desired to deodorize the interior of the vehicle it is sufficient to exert a minimum pressure against the bottom of said bellows container 1 so as to press the deodorant liquid stored therein. At this point, under the effect of the exerted pressure, a portion of the liquid will tend to come out and it will be forced through the nebulizer unit 3. The liquid drops passing through said nebulizer unit 3 then will soak the inner porous wall of said diffusing element 7, which slowly with time will provide for diffusing the scent of said drops. In order to facilitate the operation of pressing said container 1, fig. 2 shows a second preferred embodiment of the dispenser according to the present invention which has, in respect of the embodiment of fig. 1, a surface 8 supporting the fingers of the user, which predictably will use the thumb in order to exert the aforesaid pressure. The function of said cylindrical body 6, fitted on said container 1, is therefore to limit the stroke of said bellows in order to avoid consequently an excessive amount of deodorant liquid from being delivered by user, that is the amount the diffusing element 7 is fully soaked by.

## Claims

1. A deodorant liquid dispenser (10) comprising:
(i) a bellows container (1) for said deodorant liquid integral with a
(ii) cylindrical end (2) housing a
(iii) nebulizer unit (3),
(iv) a diffusing element (7) downstream said nebulizer unit (3) said diffusing element (7) absorbing drops of said nebulized deodorant liquid from said nebulizer unit (3) under the pressure exerted by said bellows container (1) when in its pressed configuration.

2. The dispenser (10) according to claim 1, wherein said nebulizer unit (3) comprises a nebulizer (4) and a section reducer (5).

3. The dispenser (10) according to claim 1, wherein said diffusing element (7) is constituted by a body made of porous material for absorbing drops of said nebulized deodorant liquid.

4. The dispenser (10) according to claim 3, wherein said diffusing element (7) further comprises a finger supporting surface (8).

5. The dispenser (10) according to claim 4, wherein a cylindrical body (6) is fitted onto said container (1) in order to limit its stroke during the pressure exerted by user.
